# EUROPEAN PATENT SPECIFICATION

(11) **EP 0 796 276 B1**
(45) Date of publication and mention of the grant of the patent: **15.10.2003**
(21) Application number: 95929258.2
(22) Date of filing: 30.08.1995
(51) Int. Cl.: C07K 14/54, C07K 1/107, C12N 15/24, A61K 38/20

(54) **GRADUAL MODIFICATION, SUPER-AGONISTS AND ANTAGONISTS OF SIGNAL-PROTEINS AND PEPTIDES**
PROGRESSIVE ANDERUNG VON SIGNAL-PROTEINEN UND PEPTIDEn UND DIE HERGESTELLTEN SUPERAGONISTEN UND ANTAGONISTEN
MODIFICATION GRADUELLE, SUPER-AGONISTES ET ANTAGONISTES DE PEPTIDES ET DE PROTEINES-SIGNAUX

(30) Priority: 31.08.1994 NL 9401404; 10.05.1995 NL 1000332
(43) Date of publication of application: 24.09.1997
(73) Proprietor: PHARMA KEY B.V., 9736 AM Groningen (NL)
(72) Inventor: SMIT, Victor, 9736 AM Groningen (NL); HUPPES, Willem, NL-3055 EG Rotterdam (NL)
(74) Representative: van Westenbrugge, Andries
(86) International application number: NL9500292
(87) International publication number: WO96006860

(56) References cited:
- WO-A-89/05824
- ELECTROPHORESIS, vol.15, no.2, , WEINHEIM pages 251 - 254 V.SMIT 'Native electrophoresis to monitor chemical modifications of human Il-3'
- PROCEEDINGS OF THE NATIONAL ACADEMY OF SCIENCES OF USA, vol.89, 2, WASHINGTON US pages 11842 - 11846 A.F.LOPEZ ET AL 'A hIL-3 analog with increased biological and binding activities' cited in the application
- JOURNAL OF BIOLOGICAL CHEMISTRY, vol.266, no.16, 5 June 1991, MD US pages 10624 - 10631 N.A.LOKKER ET AL 'Structure-activity relationship study of hIL-3' cited in the application
- L.STRYER 'biochemistry' , FREEMAN , NEW YORK see page 162; figures 7-38

## Description

### Scientific field of the invention:

This invention is on the area of the chemical modification technology of biologically active proteins and peptides. More specifically it concerns use of chemical modification to obtain a protein or peptide with superior properties or with new or even counteracting properties. In addition, the invention also concerns a new method for structure-function analysis by using gradual chemical modification, a biological principle, namely the catalytic activity of signal peptides and the successful abrogation thereof, leading to a very effective inhibitor of Acute Myeloid Leukemia cells.
As illustration, but not as limitation, we show the chemical modification of human IL-3, a protein that also has a substantial therapeutic value after modification. This patent-description also contains specific examples within the therapeutic field of the invention.

### Field of the applications:

There are two possible fields of applications that are important for the invention: A field for an IL-3 with superior properties (Superagonist) or a field for an IL-3 with counteracting or new properties (Antagonist). In this patent description Superagonists are for instance an IL-3 with a lowered antigenecity and/or a higher biological activity and/or a higher stability.
Possible applications of IL-3 Superagonists are:
- reduction of the cytopenic phase after myelo-ablative therapy like after inductive therapy for bone marrow transplantation or after accidental radiation.
- induction of a synchronized cell-cycle of cells with an IL-3 receptor, for instance for chemotherapy of leukemia's.
- induction of enhancement of the IL-3-dependent progeny both for the number of cells and their activation, for treatment of diseases like worm infections, tuberculosis, fungal infections and certain viral infections.
- selective outgrowth of the bone marrow towards nucleus containing cells except lymphocytes, for instance with burning wounds and non-homologous skin-transplants.

Some, but not all examples of applications of signal-substance antagonists (with counteracting or cell-inhibitory activity), more specifically of IL-3 are:
- inhibition and/or neutralization of myeloid cells in bone marrow transplantation.
- myelo-suppression in auto immune-diseases. cancer and diseases of the blood forming organs, like sickle cell anemia and thallisemia.
- treatments to cure all sorts of cancer that involve cells with the IL-3 receptor, more specifically almost all forms of acute myeloid leukemia or chronic myeloid leukemia, B-cell lymphoid tumors or other forms of cancer that are stimulated by IL-3, for instance certain follicle cell tumors.
- induction of self-tolerance towards the tissues in auto immune diseases like arthritis, rheumatic arthritis and diseases of the central nervous system, by suppression or elimination of lymphoid cells that contain the IL-3 receptor. This can also lead to the impaired generation and elimination of effector cells like the eosinophilic granulocyte. Finally it is possible that there is a direct interaction with these cells, thus enabling a direct cure of the eosinophilic syndrome. This is also of great importance in acute phases of worm infections and hypersensitivity reactions to for instance medicinal drugs.
- curing eosinophilic syndromes like eosinophilic gastritis and enteritis, fascitis, granulomatosis, sinusitis, pneumonia, asthma, Churg Strauss syndrome and other angitis treatments of the shock syndrome for instance by killing or suppressing the number of effector cells.
- ablation or suppression of cells with an IL-3 receptor, like lymphoid cells and/or effector cells like the eosinophilic granulocyte, for the treatment of allergies. In these cases both suppression of the allergy and induction of tolerance towards the antigen are possible.
- other allergic reactions where the action of IL-3 is involved.
- treatments to prevent metastases that are stimulated by IL-3 mediated adhesion.
- treatment of infectious diseases, for instance by suppressing an acute phase where there is an occurrence of excessive amounts of growth factor in the blood stream.

One or more of these applications can also be mentioned for other growth factors like the other Interleukins 1-8, GM-CSF, TNF and gamma IFN, which are also good candidates for modification according to this invention. Hereby 'IL-3' is to be replaced by the other signal substance. Since the modes of interaction of the various substances in the various diseases can differ from substance to substance, several synergistic effects can also be expected. Therefore these are also ebodied in the invention. Finally, the following applications can be added or specified:
- IL-1 inhibition to suppress IL-1 stimulated metastases of melanoma's and forms of lung cancer.
- IL-1 inhibition to suppress Alzheimer's disease
- IL-2 inhibition to suppress capillary leak syndrome
- IL-2 inhibition to suppress periodontitis
- IL-4 inhibition to suppress periodontitis
- IL-4 inhibition to suppress IL-4 stimulated viruses like for instance the radiation leukemia virus in mice.
- IL-5 inhibition to suppress IL-5 stimulated respiratory tract infections.
- IL-6 inhibition to suppress Rheumatoid arthritis.
- IL- 10 inhibition to suppress infections of Mycobacterium.

Since it is perceptible that the modified signal peptides can also be generated by molecular biology, these mutant proteins, DNA-constructs and the use thereof are also considered to be within the scope of the invention. Hereby, one can include gene-therapy with the constructs that contain the code for such peptides and/or proteins. The use in gene-therapy can result in cells that are producing and excreting the super-agonist or the antagonist. Therefore, in one of the examples there is also an elaboration on the possibility to construct a growth factor with a reduced stability. This can be used especially in combination with antagonistic action, thus resulting in a selective administration whereby the high rate of breakdown of the antagonist can contain the action to a very localized environment. This is particularly interesting in gene-therapy for solid tumors. If there is such a cell in or near the tumor, especially this tumor will experience the exposure to the maximal effect. If there is an additional instability of the produced signal peptide, the effect of the peptide can be very localized, thus leading to the expectation of fewer side-reactions. In one of the examples it is demonstrated that it is possible to generate a signal peptide with a lowered stability. Since such peptides can also be generated with deletion and/or substitution mutants, such gene-therapeutic applications and gene therapy- constructs are also considered to be within the field of applications of the invention.

This patent-description also elaborates on the use of chemical modification, protease treatment and mass spectrometry. This can be applied to every modification of every peptide or protein, provided it can be specifically fragmented for instance with protease's. The quantitative structure-function analysis part of the description contains the successful use of the combination of gradual chemical modification, protease treatment and laser desorption mass spectrometry with biological assays. Therefore, the application of this analysis on any peptide and/or protein that can be modified, specifically fragmented and is biologically active is also considered to be within the scope of the invention. It is also possible to use other mass spectrometric techniques like electrospray mass spectrometry, which is particularly suitable after for instance exoprotease treatment.

Finally, the description of the invention also discloses the importance of metal ions with more than 1 valence in charge, by preference Zinc-ions, especially for catalytic activity and the subsequent efficacy. Therefore, it is possible to influence the growth factor action by manipulating the (local) metal ions concentrations. Since there is a usual optimum in efficacy of the metal ions concentration, and any concentration below or beyond this optimum can lead to reduction of efficacy of the growth factor. This enables manipulation of the efficacy of the growth factor. In this way also indirect therapeutic effects can be achieved with metal-, preferably Zinc ions. This can be used in treatment of skin-diseases like wrath's in the form of ointments. Also, the use of inhalation-sprays for treatment of lung-affects is possible.

### Background of the invention:

### Human Interleukin-3:

Interleukin-3, which was first isolated from T-cells as a glycoprotein, has been demonstrated to act on bone-marrow cells. Either with or without other growth factors it has been shown to induce the formation of various blood cells from these bone-marrow cells. Human IL-3 has been cloned for the first time in 1987 by Dorssers et al. who used a human c-DNA library and hybridization with a probe of mouse DNA (Gene 55:115 (1987)).

### Structure-function relationship of human Interleukin-3:

Several articles have been published concerning the structure-function relationship of human Interleukin-3 (J.Biol.Chem. 266: 21310 (1991); J.Clin.Invest 90:1879 (1992); J.Immunol. 146:83 (1991); EMBO J. 10:2125 (1991); Proc.Natl.Acad.Sci. USA 89: 11842(1992)).
In their research they all used molecular biological techniques like the generation of deletion and substitution mutants. In the case that substitution mutants were used, the choice was based on the amino-acid homology of IL-3 with other species (mouse, rhesus monkey, gibbon) or it was based on deliberate changes of polarity or structure. With deletion mutants the protein was scanned for biological importance by removal of parts of the protein. Because of practical problems with regard to purification of the mutant-proteins (muteins) these mutant proteins have never been checked for major structural changes. This is a major problem since these structural changes usually do occur. As described, sometimes they are even deliberately introduced. As a result any statement regarding the involvement of various amino acids on the biological activity can only be made with the greatest reservations. In WO-A-89 05824 a variant of IL-3 is described obtained by a combination of site-directed mutagenesis and chemical modification. The lysine residues at amino acid positions 10, 28, 100, 110 and 116 are replaced by arginine residues and the lysine residues 66 and 79 are coupled to polypropylene glycol. Electrophoresis 15:251 (1994) describes the chemical modification of hIL-3 by acetic anhydride or succinic anhydride at pH's of between 5.5 and 9.5 to demonstrate the improved quality of separation of a new electrophoresis buffer system.

Present invention has a different approach. The native IL-3 is used as starting material. By using stepwise gradually increasing modification an extra selectivity is introduced in the chemical reaction. The modification that occurs simultaneous with the first change in biological activity can be simply localized with specific proteases and new forms of mass spectrometry. As a result, important amino acid residues can be quickly localized and minimal change (and therefore maximal control) can be achieved in the introduction or change of a desired property. There is no need to purify the modified material and thus there are no problems in losses during purifications. This again enables an easy verification of the secondary structure. Therefore this invention is an improvement as compared to molecular biological techniques because the approach is more convenient, better verifiable and faster than the molecular biological approach.

### Modified growth factors:

### Superagonists:

In many structure-function research efforts on the various growth factors, muteins have been found that have an improved activity. For instance in the European patent application EP-131816 the described goal is to obtain a beta-Interferon with an improved biological activity and/or a less side-reactivity. Also various examples of chemical modifications are available: for instance the European application EP 236987 describes the modification of IL-2 to obtain a less toxic and less immunogenic substance with improved kinetics in clearing from the body. Patent application EP-0442724 describes PEG-ylated IL-6 which is a product with a longer half-life and an enhanced biological activity. Patent application WO 88/01511 describes succinylation of IL-2 whereby an enhanced solubility is achieved. In non of these patent applications there is any modification strategy besides a trial and error approach in which random mild modification is used to obtain one or a few modifications on the molecule. In addition, substantial losses in purification can occur, which was also the case in the example of PEG-ylation of IL-6 where only a 10 % yield of the desired protein was obtained. The localization of the modification was done in none of these inventions.

In present invention there is almost no loss and the localization of the modification can well be performed. In addition, gradual chemical modification makes it even possible to specifically modify 1 residue at 1 place in the molecule, as demonstrated in the examples 1 and 2. Although most modifications are performed with irreversible agents, also reversible agents can be used. This also enables the very specific modification of other residues.

### Antagonists:

In the European patent application EP-0413383A 1 an antagonistic effect of a human IL-3 mutant is mentioned. However in this case it concerns the relation of remaining biological activity as compared to its receptor binding capacity. Thus, a real suppression the activity of the native IL-3 was not shown.
In patent applications PCT/US93/11197 and PCT/US93/11198 al kinds of IL-3 mutants are claimed, but also in this case there is no support what so ever of a real inhibitory activity. In addition, it is not probable that the mutants with the lowest activity are also the best inhibitors, since the chance on structural distortion is much higher than the chance on specific elimination of the catalytic activity.
There are other real antagonists for receptors of bovine growth hormone (Endocrinology 130:
2284 (1992)), mouse interleukin-2(EMBO J. 11: 3905 (1992)), human hepatocyte growth factor (Biochemistry 31: 9555(1995)), IL-1(Scand. J. Immunol. 36: 27(1992)) and IL-4 (J. Exp. Med. 178: 2213 (1993). In all these publications no modification strategy was described; the antagonists were all by-products of structure-function analysis research. In addition, the concentrations of inhibitor that were needed to obtain significant inhibition were on average a hundred fold higher than the native growth factor concentration. These are concentrations that are harmful to clinical value of these antagonists.
The only strategy to generate a growth factor antagonist has been used for human growth hormone (Science 256: 1677(1992)). This was based on the disruption of one of the two receptor binding sites of the hormone. Also in this case there was a decrease of a factor 50 in receptor binding capacity, therefore requiring a problematic excess of inhibitor as compared to growth factor.

However, with present invention it is possible to obtain a clinically applicable inhibition, possibly even with an enhanced receptor binding capacity. This can be explained with the hypothesis that the growth factor itself performs some kind of catalytic activity after receptor binding. This hypothesis was supported by the fact that IL-3 contains a catalytic zinc-ion (Biochem. Biophys. Res. Commun. 187: 859 (1992)). It is not necessary that the growth factor contains complete catalytic center. It is very well possible that the catalytic center is completed only in the growth factor -receptor binding. Therefore the concerning chemical modification is directed as specific as possible towards residues that are directly or indirectly involved in such Zinc binding and/or catalysis and this without distortion of receptor binding ability. The starting material can be any protein or peptide containing substance. However, because the Zinc ions can protect against denaturation, it might be necessary to reversibly denaturate the molecule and to add chelating agents to remove this Zinc from the molecule. As an illustration, but not as an limitation of the invention the modification of IL-3 with lodo-acetate is described. At the concerning pH this modification is directed to alkylation of His-residues. The method however, can be performed easily also with other reagents by a person that is skilled in the art. The same can be stated about the modification of other amino acids that are involved in the catalytic activity and/or Zinc binding. Also these residues can be easily modified by other chemical ways or by molecular biological ways, for instance by using deletion or substitution mutations. In addition this is not limited to IL-3 alone: There is a significant homology among the various receptors of the cytokine-superfamily: For instance Interleukins 2-7 Epo and GM-CSF. On top of that specific Zinc binding has also been found for IL-2 IL-6 GM-CSF and gamma IFN. It is also conceivable that the invention can be applied to much more signal peptides and/or proteins, since for instance Insulin, human growth hormone and prolactin also have specific Zinc binding properties. In addition, it has been found that for certain cell-lines IL-3 can be replaced by for instance Insulin. Finally it is possible that alkylation of a growth factor also has an other mechanism in the generation of a antagonist or cell-inhibitor. Therefore, in general also alkylation, preferably with Iodo-acetate, is to be regarded as a separate approach in the invention. Therefore all the above mentioned applications and/or modified substances and/or DNA-constructs and the use thereof are embodied within the scope of the invention.

### Examples:

### Example 1:

Gradual chemical modification of IL-3 with Acetic Anhydride to obtain an IL-3 with an enhanced biological activity or a changed stability.

### Materials and Methods:

### Chemical Modification:

Acetic Acid, dioxane, lysine hydrochloride and MES were from Sigma. the modifying agent was from Fluka.
Buffers: Acetate/NaOH was used for modification at pH 5.0, MES/NaOH was used for modification at pH 5.5, 6.0 and 6.5. At pH 7.0 an NaH₂PO₄ / NaOH buffer was used. Ten times concentrated stock solutions were prepared and directly filtered trough a 0.22 micron filter.

The reaction mixture contained 50 mM buffer, 2 mg/ml hIL-3 and 3 mM Acetic anhydride or Succinic anhydride respectively. The 10 times concentrated stock solutions were prepared fresh at the day of the experiment. Modification of hIL-3 was performed overnight at 30 °C. After modification it was determined by SDS-electrophoresis that the IL-3 was not degraded.

### Tests of biological activity:

MO-7 cells were a kind gift of dr. I.P. Touw (Erasmus University of Rotterdam, The Netherlands). RPMI culture medium was from Gibco (Paisly, UK.). supplemented Bovine Calf Serum was from Hyclone (Logan, Utah, USA.) The cell culture medium consisted of RPMI with 10 % of the calf serum. During normal tissue culture of the cells also 100 ng/ml IL-3 was added.

Firstly 10 fold stock solutions were prepared , consisting of serial 3 fold dilution's ranging from 10 microgram/ml to 1 ng/ml in cell culture medium. after mixing thoroughly, 25 microliter of the stock solution was added to 225 microliter cell culture medium, followed by incubation at 37 °C. For the 6-day tissue culture 2 x 10⁵ M07 cells /ml were used as an end concentration and for the 10 day culture 4 x 10 ³ M07 cells/ml were used. After the tissue culture, overnight tritium thymidine incorporation was used to determine biological activities. From at least 2 independent growth-response curves there was a determination of the average (and range of ) the concentrations that resulted in 50 % maximal stimulation. The relative activity of the modified substances was determined as the ratio of these 50 % concentrations of the modified and the native IL-3 respectively ( [IL-3_{modified}]^{50%} / [IL-3ₙₐₜᵢᵥₑ]^{50%}). The standard activity for the native IL-3 on day 6 was 1.0 million units/mg of protein (n=10, sigma₍ₙ₋₁₎ = 20 %). On day 10 it was 0.2 million units/mg of protein (n=10, sigma₍ₙ₋₁₎ = 30 %).

### Results:

All results regarding to the more precise characterization in terms of average number of groups, the specificity and the places of the modification on the molecule are described later in this patent description. The results of the biological tests are shown in table 1:

**Table 1:**

| Relative biological activities of acetylated IL-3. | | |
|---|---|---|
| Acetylation at culture | Average relative activity (and activity-range) in tissue | |
| | After 6 days | After 10 days |
| pH = 5.0 | 1.5 (1.4-2.1) | 1.4 (0.8-1.5) |
| pH = 6.0 | 0.9 (0.8-1.0) | 0.9 (0.7-1.1) |
| pH = 6.5 | 0.5 (0.4-0.6) | 0.2 (0.2-0.3) |
| pH = 7.0 | 0.9 (0.9-0.9) | 0.6 (0.6-0.6) |

From the table it can be concluded that there is a significant difference in relative activity between day 6 and day 10 after modification at pH 6.5 and pH 7.0. Therefore, it can be assumed that this demonstrates the generation of a substance with significantly lowered stability. At pH 5 there might be an enhancement of biologic activity. Further aspects are discussed in examples 5 and 6.

### Example 2:

Gradual chemical modification of IL-3 with Succinic anhydride for the generation of an improved IL-3 with an enhanced activity or stability.

### Chemical Modification:

Acetic Acid, dioxane, lysine hydrochloride and MES were from Sigma. the modifying agent was from Fluka.
Buffers: Acetate/NaOH was used for modification at pH 5.0, MES/NaOH was used for modification at pH 5.5, 6.0 and 6.5. At pH 7.0 an NaH₂PO₄ / NaOH buffer was used. Ten times concentrated stock solutions were prepared and directly filtered trough a 0.22 micron filter.
The reaction mixture contained 50 mM buffer, 2 mg/ml hIL-3 and 3 mM Acetic anhydride or Succinic anhydride respectively. The 10 times concentrated stock solutions were prepared fresh at the day of the experiment. Modification of hIL-3 was performed overnight at 30 °C. After modification it was determined by SDS-electrophoresis that the IL-3 was not degraded.

### Results:

All results regarding to the more precise characterization in terms of average number of groups, the specificity and the places of the modification on the molecule are described later in this patent description. The results of the biological tests ( as performed in example 1) are shown in table 2:

**Table 2:**

| Relative biological activities of Succinylated IL-3. | | | | |
|---|---|---|---|---|
| Succinylation at | Average relative activity (and activity-range) in tissue culture | | | |
| | After 6 days | | After 10 days | |
| pH = 5.0 | 1.7 | (1.6-2.0) | 1.6 | (1.3-2.5) |
| pH = 6.0 | 1.4 | (1.2-1.8) | 1.3 | (1.3-1.4) |
| pH = 6.5 | 0.4 | (0.4-0.5) | 0.4 | (0.4-0.5) |
| pH = 7.0 | 0.3 | (0.3-0.3) | 0.3 | (0.2-0.3) |

From table 2 it can be concluded that succinylation at pH 5 results in a significant enhancement of the activity and succinylation at a pH => 6 results in a lower activity.

### Example 3:

A method to chemically modify biologically active peptides or proteins for the generation of a protein or peptide antagonist.

### Materials and methods:

Urea, EDTA, MES and NaOH were from Sigma. the Na-Iodo-acetate was from Fluka.
Buffers: MES/NaOH was used for modification at pH 6.0. Ten times concentrated stock solutions were prepared in 8 M urea and filter-sterilized directly thereafter with a 0.22 micron filter. The reaction mixture contained 50 mM buffer, 5.5 M urea and 50 mM EDTA. From these reagents 10 times concentrated stock solutions in 8 M urea were made freshly on the day of the experiment.

### 1. Moderate chemical modification of hIL-3:

lodo-acetate was added in a concentration of 3, 10 and 30 mM. The IL-3 concentration was 2 mg/ml. The modification was performed during 24, 48 and 72 hours at 37 °C and subsequently it was studied by native electrophoresis. After 2 days and 30 mM Iodo-acetate there was maximal modification without severe distortion of the bands from the modified material (an indication of severe denaturation of the molecules). In this case less than 2 % of the starting material was left. Because in this case there was an expectation of a minimal biological activity without severe denaturation of the protein, this sample was used in further experiments. Subsequently, SDS electrophoresis was used to demonstrate that no degradation of the molecule was found after modification.

### 2. Partial chemical modification:

In order to optimize the inhibitory capacity of the modified hIL-3, also partial modification was performed. For this purpose 1 mg/ml IL-3 was modified for 18 hours at 37 °C with 10, 30 and 100 mM of lodo-acetate. After native electrophoresis and coomassie staining the sample of 100 mM gave maximal modification without excessive distortion of the bands in the gel. circa 5 % of the starting material was still present. Since only this sample showed inhibitory activity in the activity tests, this sample was used in further experiments.

### 3. The activity- and inhibition assays:

The activity tests were performed as described in example 1. Growth response curves (n>= 2) for both controls and alkylated IL-3 were made by 10 fold serial dilution's ranging from 1000 to 1 ng/ml. Inhibitory activity of the alkylated IL-3 was tested by performing the same titration of control IL-3, but now in presence of 3 ng/ml alkylated IL-3.

In order to determine the maximal receptor binding capacity partially modified IL-3 was titrated in serial 7 fold dilution in presence of 3 ng/ml of native IL-3. The titration range was from 15µg/ml to 0.1 ng/ml and the titration was performed on only 4000 M07 cells/ml to exclude any starvation phenomena's.

### Results:

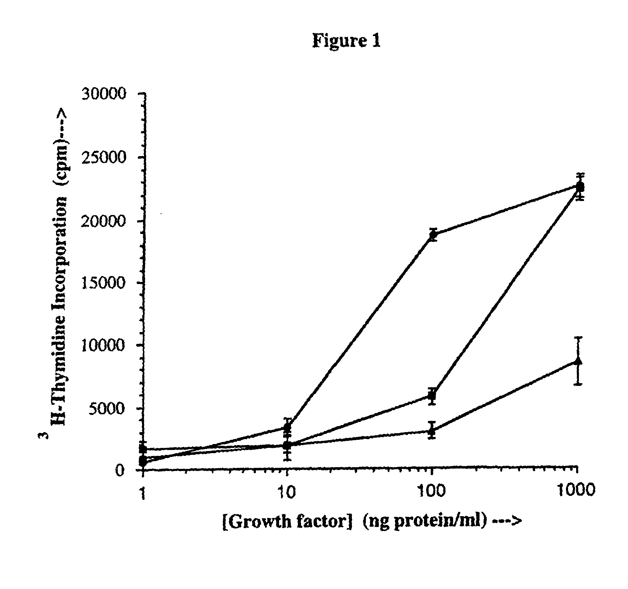

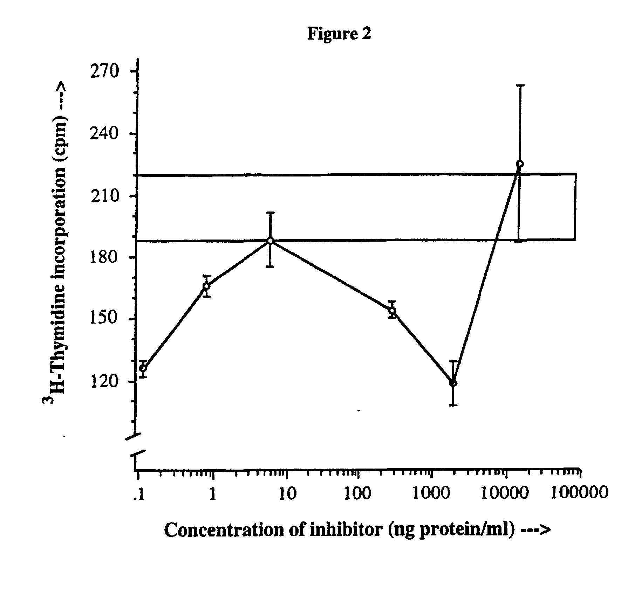

In figure 1 it is shown that the modified IL-3 is capable to inhibit the control-IL-3 by a factor 10-100. In addition 3 ng/ml of the modified IL-3 is able to suppress thymidine incorporation of 30-100 ng/ml control IL-3 for 80-90 %. Therefore, the modified IL-3 does not only have an Inhibitory activity, it also has an enhanced receptor binding capacity. This is confirmed in the titration of partially modified IL-3 (figure 2): Only 0.1 ng of partially modified IL-3 is sufficient for almost 50 % inhibition of 3 ng/ ml native IL-3.

### Example 4:

Method for gradual enzymatic exoprotease treatment of IL-3 for the generation of a modified IL-3 with a changed stability and/or activity.

### Materials and methods:

Exoprotease treatments have been performed with Cathepsine-C and with Carboxypeptidase-Y from Boehringer. 1 mg/ml of IL-3 was incubated for 18 hours at 37 °C in presence of the protease. Cathepsine-C was added in serial two fold dilution's in a range of 1/2 to 1/128 mg/ml. The other reaction conditions were as described by the manufacturer.
Biological activities have been determined as described in example 1.

### Results:

The approach lead to the results in table 3. The modifications that did not result in a change in biological activity are not shown:

**Table 3 :**

| Changed activities after gradual exoprotease treatment: | | | | | |
|---|---|---|---|---|---|
| Protease treatment culture | [Protease] | Average relative activity ( and range) in tissue | | | |
| | (mg/ml) | After 6 days | | After 10 days | |
| | | | | | |
| Carboxypeptidase Y | | | | | |
| | 1/40 | 0.6 | (0.5-0.7) | 1.0 | (0.7-1.5) |
| | 1 / 20 | 0.08 | (0.07-0.10) | 0.2 | (0.13-0.21) |
| | 1/10 | <0.05 | (<0.05) | <0.05 | (<0.05) |
| | | | | | |
| Cathepsine C | 1/32 | 2 | (2-6) | 1.0 | (0.7-1.5) |
| | 1/16 | 5 | (3-6) | 1.0 | (0.9-1.1 ) |
| | 1/8 | 3 | (2-4) | 1.3 | (0.9-1.7) |
| | 1/4 | 3 | (2-4) | 1.3 | (0.9-2.1) |

From this table it can be concluded that treatment with Carboxypeptidase Y at a concentration of 1/40 and at 1/20 results in a substance with a relative activity that is lower at day 6 than at day 10. Therefore, this indicates a substance with a higher stability as compared to native IL-3.
It can also be concluded form the table that Cathepsine C significantly enhances the activity at day 6 but not at day 10. Therefore, this is a also substance with a lowered stability.

### Example 5:

Localization of modifications in a peptide or protein by the combination of protease treatment and mass spectrometry.

### Materials and methods:

### Protease treatment:

For localization of the modified residues the modified material was dialyzed against the appropriate buffer and subsequently fragmented by Endo Glu-C or Endo Lys-C as described by the manufacturer (Boehringer Mannheim, Germany). The incubation was overnight at 37 °C, 2 mg/ml hIL-3 and a protein to protease ratio of 30.

### Laser Desorption Mass Spectrometry (LDMS):

### Pre treatment:

Solutions of 2,5 dihydroxybenzoic acid (DHB, Mᵣ=154.12; 10 g/l) in milli Q water were made freshly before each experiment. Both the (un)modified IL-3 solutions and their digests were diluted to 0.1 mg/ml. From these diluted solutions 0.5 µl was mixed with 0.5 µl DHB-solution on the target. Subsequently, the target was dried to air at room temperature in a slow stream of air.

### Mass Spectrometry:

Matrix Assisted Laser Desorption mass spectrometry was performed on a Finnegan MAT Vision 200 laser desorption mass spectrometer, equipped with a pulsed nitrogen laser (337 nm, pulse width 3 ns). The sample was exited to just above the ionization threshold (10⁶-10⁷ W/cm²). The acceleration voltage was 6.5 kV. The ions were post accelerated to the conversion dynode on - 10 kV for the electron amplification. Standard accuracy was about 0.05 %, but this can deteriorate to 0.1-0.2 % due to experimental conditions.

### Results:

Because the signal of the LDMS was still sufficient in spite of the higher molecular weights, it became possible to localize all modifications. Two examples are shown:
1- Localization of modification with Succinic anhydride (pH 5.0) by means of Endo Lys-C treatment and LDMS:
   At the pH = 5.0, Succinic anhydride modification and subsequent Endo Lys-C digestion a peak shifted from 1085 d to 1185 d, also the peak at 1108 d (1085 + 23 from Na⁺) shifted to 1208 d. Based on the protease specificity and the amino acid sequence this 1085 d peak can only correspond Ala¹ - Lys¹⁰. Since modification of Lys¹⁰ would disable the digestion on this amino acid, there would not be any Ala¹ - Lys¹⁰ -fragment at all. Therefore, the modified amine residue is the amino terminus Ala¹.
2- Detection of modification of Lys²⁸ with Acetic anhydride by both Endo Glu-C treatment with subsequent LDMS and Endo Lys -C treatment with subsequent LDMS:
   At the pH 7 Acetic anhydride modification and subsequent Endo Glu-C treatment the peak at 1598 d shifted towards 1640 d. This shift corresponded exactly with the mass of 1 acetyl group. Also in this case the amino acid sequence enabled the localization to IIe²³ - Asp³⁶ . Since Lys ²⁸ is the only amine-residue in this fragment, it can be deduced that this is the modified residue. This was confirmed by the Endo-Lys digestion where a fragment of 5815 d emerged. This fragment can only be explained if Lys²⁸ is the modified residue, thus disabling the digestion after that residue.

The other modifications have been analyzed in a similar way, resulting in table 4:

**Table 4:**

| Localization of modifications on IL-3: | | | | |
|---|---|---|---|---|
| Number of modified groups after modification with | | | | |
| Modified at pH: | Acetic anhydride | | Succinic anhydride | |
| | | | | |
| 5.0 | Ala¹ | >90 % | Ala ¹ | >90% |
| | | | | |
| 6.0 | Ala¹ | >90 % | Ala ¹ | >90% |
| | Lys ²⁸ | ±45 % | Lys ²⁸ | ±45 % |
| | Lys ⁶⁶ | ±20 % | Lys ⁶⁶ | ±20 % |
| | Lys ¹⁰⁰ | ±40 % | Lys ¹⁰⁰ | ±25 % |
| | Lys ¹¹⁶ | ±40 % | Lys ¹¹⁶ | ±40 % |
| | | | | |
| 6.5 | Ala¹ | >90 % | Ala ¹ | >90% |
| | Lys ²⁸ | ±70 % | Lys 28 | ±55 % |
| | Lys ⁶⁶ | ±50 % | Lys ⁶⁶ | ±40 % |
| | Lys ¹⁰⁰ | ±65 % | Lys ¹⁰⁰: | ±50 % |
| | Lys ¹¹⁶ | ±80 % | Lys ¹¹⁶: | ±90 % |
| | | | | |
| 7.0 | Ala¹ | >90 % | Ala ¹ | >90% |
| | Lys ¹⁰ | ±20 % | | |
| | Lys ²⁸ | ±55 % | Lys²⁸ | ±70 % |
| | Lys ⁶⁶ | ±35 % | Lys⁶⁶ | ±40 % |
| | Lys ¹⁰⁰ | ±65 % | Lys ¹⁰⁰ | ±70 % |
| | Lys ¹¹⁶ | ±40 % | Lys ¹¹⁶ | ±90 % |

The table shows that both modifications have the same target residues on the protein. The only exceptions are that Acetic anhydride has a slightly higher degree of modification at pH >= 5.5 and at pH 7 Lys¹⁰ was partly modified in contrast to the Lys ¹⁰ of the Succinic anhydride modified material.
Table 4 also shows that Lys¹¹⁶ is at least partly protected at pH = 7. Since a phosphate buffer was used at that pH, the possibility arises that a phosphate group is binding at that place and thereby shields the Lys¹¹⁶ for modification. To test this hypothesis hIL-3 was modified at 50 mM buffering substance, consisting of MES and Phosphate. Acetic anhydride (1, 2 and 3 mM respectively) was used for the modification at pH 6.8 which is well within the buffering range of both buffers. In presence of 10 mM or more phosphate there was protection of 1 group: Lys¹¹⁶. At a phosphate concentration below 1 mM this protection was absent. Since 10 mM is the physiological phosphate concentration, it can be assumed that present localization method enabled the demonstration and localization of a biologically significant phosphate binding. Therefore it is very conceivable that an antagonist or cell-growth inhibitor can be generated by distortion of this phosphate binding. Therefore, also this is to be considered within the scope of the invention. It can also be stated that the residues Lys²⁸ and Lys⁶⁶ also had slight protection by the phosphate, suggesting a close proximity in the 3-D structure. Thus, in this way it can even provide structural information.
Finally it can also be stated that the gradual chemical modification can be performed with such minimal degree that a specificity can be accomplished that is not limited to some types of residues, not to only amine-residues, but even to 1 amine-residue on the complete molecule, namely Ala¹. Therefore this specificity is also included in the claims.

### Example 6:

Quantitative structure function analysis research using gradual chemical modification, protease treatment and laser desorption mass spectrometry.

### Materials and method:

### QSAR-strategy:

This example was demonstrated with lysine modifications on hIL-3. The strategy consists of 5 steps of which the first step concerns the gradual chemical modification of the protein. Although the micro-environment of the various residues in the 3-D structure is not known, differences can be expected on the amino acid sequence alone. Even more differences can b expected in the 3-D structure.

We investigated acylation reactions on hIL-3 (step 1). These reactions only take place on uncharged Lys residues, enabling the gradual modification by a stepwise increase in the pH of the modification-reactions.

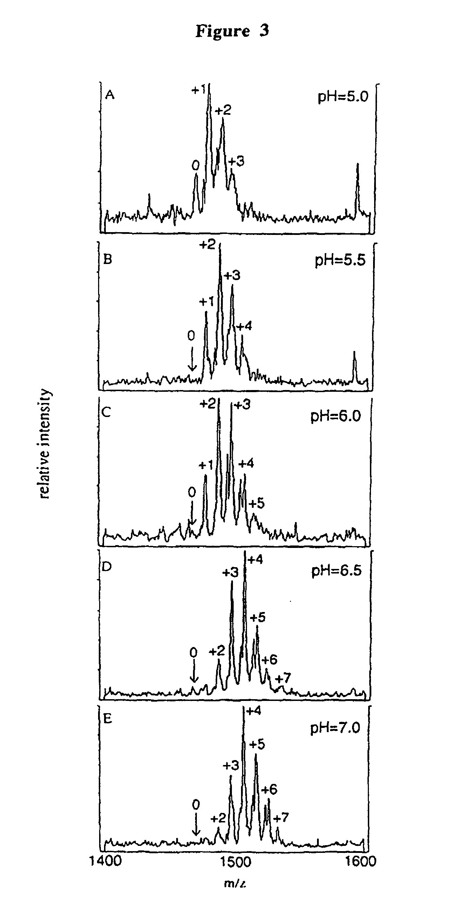

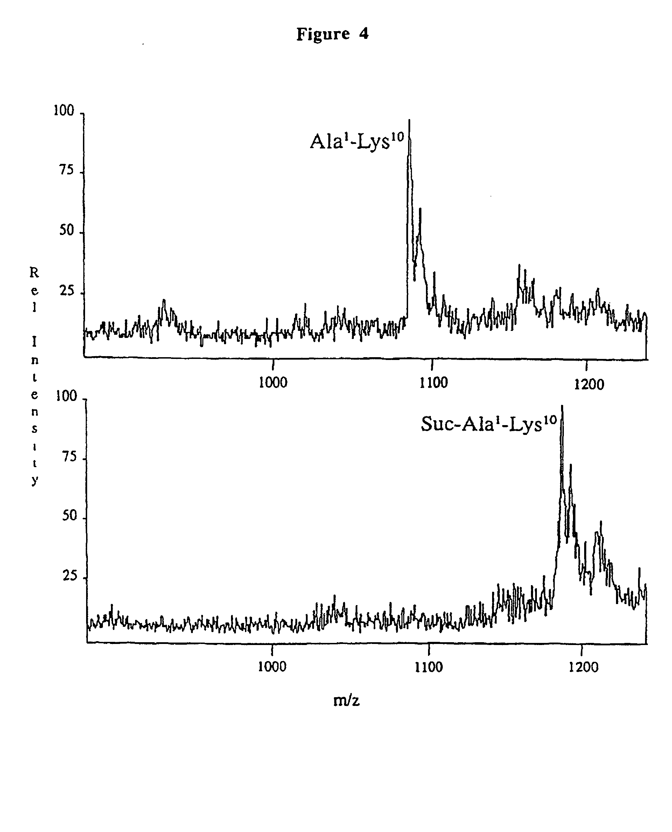

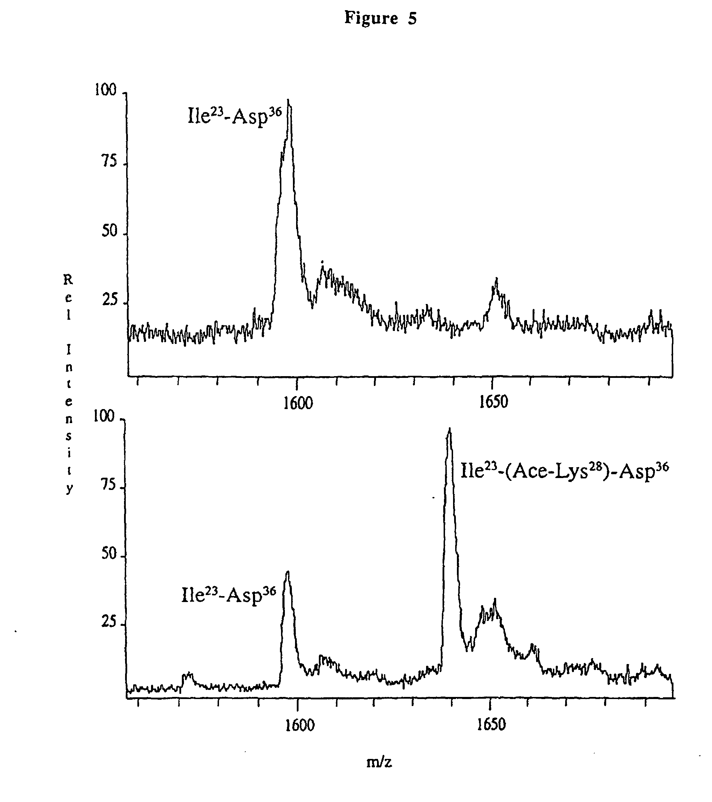

The second step is the monitoring of the modification reaction. In order to study a sufficient number of possible conditions so that the optimal conditions can be achieved, a mild and sensitive method is needed. This method is native electrophoresis (Electrophoresis 15: 251 (1994)). However, also electrospray mass spectrometry can form a suitable alternative. This is demonstrated in figure 3: Electrospray Mass Spectrometry of Succinylated IL-3 at pH 5-7. Especially the combination of both enables the demonstration of complete specificity on amine-residues.

The third step is the confirmation of the overall structural integrity. Circular Dichroism Spectroscopy can be used for this purpose(Electrophoresis 15: 251 (1994)). Although small differences are not visible by this method, substantial structural changes like denaturation are clearly detected.

The fourth step is the characterization and localization of the modified residues, for which the following techniques were used: native digestion with specific protease's, electrophoresis, electrospray mass spectrometry, and LDMS. The reaction specificity was determined by the combination of native electrophoresis and electrospray mass spectrometry. Localization was performed with endoprotease's and LDMS, as described in the previous example.

The fifth and last step is the testing of biological activity of the various modified forms of the protein. After this determination of activity the real involvement of the various localized residues can be deduced.

### Results

### Chemical modification, structural confirmation and monitoring of the reaction:

Chemical modification of hIL-3 and the monitoring was performed with Succinic anhydride or Acetic anhydride as previously described. Subsequently, it was found by Circular Dichroism that Succinic anhydride modifications at a pH larger than 7 resulted in an overall structural change (denaturation). Therefore, only the modifications at or below pH 7 were used for further investigation.

### Characterization and localization of the modifications:

See previous example.

### Activity tests of the various modified forms of the protein and localization of biologically important residues:

Both the methods and the results of the tests for biological activity are described in examples 1 and 2. The combination of these results and the results of the localization of the modified residues ( Tables 1 and 2 and previous example) enables statements on involvement of several residues. Hereby, an important change is from unmodified to modified at pH 5, which is accompanied by an enhancement in activity. Other important changes are from pH 6 to pH 6.5 (activity - decrease by a factor 2) and from 6.5 to 7 (activity increase by a factor 2):
Since Succinic anhydride modification at pH 5 is accompanied by the modification of only 1 group, namely the amino terminus (Ala¹), it can be concluded that this group has some kind of limiting or regulating action. The increase in activity has also been found in structure-function research with deletion mutants.(J. Biol. Chem. 266, 21310 (1991); Proc. Natl. Acad. Sci. USA. 89: 11842(1992)), but it was never assigned to the first residue alone.
With the differences between pH 6 and pH 6.5 there is a more complex pattern: For acetic anhydride the decrease in activity of a factor 2-4 was accompanied with a modification of Lys²⁸ of 45 % to 70 % of the groups, for Lys⁶⁶ of 20 % to 50 % of the groups and for Lys¹⁰⁰ from 40% to 65% of the groups. Finally, there occurred modification on Lys¹¹⁶ of 40 % to 80 % of the groups, which is a decrease of unmodified groups of 60 % to 20 % : a factor 3 difference. Since this difference correlates exactly with the activity decrease, Lys¹¹⁶ is the best candidate for the biological activity. This is confirmed by the factor 3 lowered modification at pH 7 (in comparison with pH 6.5), that is accompanied with a factor 3 increase in activity. Therefore, Lys¹¹⁶ is important for biological activity.
This was all confirmed by modification with Succinic anhydride. In this case there was no decrease in modification for the at pH 7 modified material as compared to the at pH 6.5 modified material. Accompanying this phenomenon there was no enhancement of activity either.

Thus it has been demonstrated that the residues Lys¹¹⁶ and the amino-terminus are of biological significance, while the amino terminus seems to have an inhibitory or regulating influence, Lys¹¹⁶ seems to be important for the biological activity. In addition, Lys¹¹⁶ is also protected by phosphate, suggesting a phosphate binding by that residue. Since the residue is also important for the biological activity of the interleukin, this suggests that phosphate-binding is of importance for the mode of action of IL-3 and if this process is of importance for IL-3, it can also be of importance for other peptides and proteins.

Summarized, this method enables the localization of biologically important residues and the demonstrated phosphate binding has also enabled the establishment and localization of a possibly important physiological process. Therefore, the invention also embodies the modification of a protein or peptide to introduce a new, preferably antagonistic activity by means of the manipulation of the phosphate binding of the protein or peptide.

## Claims

1. A method for quantitative structure function analysis research of biologically active proteins or peptides selected from human receptors such as interleukins, haemopoietic growth factors, peptide hormones or protein hormones, signal peptides or signal proteins, for the introduction of one or more of the following features: enhanced biological activity, enhanced stability, suppressed antigenicity, acquired antagonistic activity or cell inhibitory activity, said method comprising applying a specific chemical modification of selected amino acids using
a) gradual chemical modification of the protein or peptide, followed by
b) monitoring the modification reaction with a mild and sensitive method such as non denaturing electrophoresis and/or electrospray mass spectrometry and said monitoring optionally further comprising confirming the overall structural integrity e.g. using Circular Dichroism Spectroscopy,
c) protease treatment,
d) mass spectrometry and
e) assaying biological activity of the modified product and optionally assaying stability of the modified product, said proteins or peptides preferably being selected from interleukins 1-8, interleukin 10, GM-CSF, TNF, insulin, prolactin and gamma IFN, more preferably GM-CSF, EPO or an interleukin being selected from interleukins 2-7 selected from the cytokine super family.

2. A method according to claim 1 wherein specific digestion with specific proteases and mass spectrometry is carried out for characterisation and localisation of the modified amino acids.

3. A method according to claim 1 or 2 wherein specific digestion with specific endoproteases and LDMS is carried out for characterisation and localisation of the modified amino acids, said endoprotease for example being Endo Glu C or Endo Lys C.

4. A method according to any of claims 1-3 wherein the modification is carried out by specific digestion with specific exoproteases and electrospray mass spectrometry is carried out for characterisation and localisation of the modified amino acids, suitably the exoprotease is N terminal e.g. Cathepsine C or C terminal e.g. carboxypeptidase Y.

5. A method according to any of claims 1-3. wherein the modification is chemical modification, said modification being alkylation and/or said modification being acylation, such as acetylation e.g. by Iodo acetate or succinylation e.g. by succinic anhydride, said modification suitably being a modification under gradually varying conditions, wherein one or more of the following conditions are varied as follows: pH between 5.0 and 7.0, preferably in steps of 0.5 pH units, and/or time or reagent-concentrations are varied.

6. A method according to claim 5, wherein the modification is carried out in the presence of phosphate buffer, preferably in combination with acetic anhydride.

7. A method according to orfe or more of the preceding claims, for the introduction of an antagonistic and/or cell inhibitory activity. wherein the modification has specificity to one or more residues that are involved in catalytic activity e.g. wherein the modification is within or in close proximity to a partial or complete catalytic center, said modification preferably changing the catalytic activity, suitably said residue is a histidine residue.

8. A method according to any of the preceding claims, wherein the modification is within or in close proximity to a metal binding center, preferably a Zinc binding center, suitably said residue is a histidine residue.

9. A method according to one or more of the preceding claims, wherein the modification is performed by reversibly denaturing the substrate and adding chelating agent to remove the metal ion e.g. in the presence of urea and EDTA, said urea preferably having a concentration larger than 5 M and said EDTA preferably having a concentration of 50 mM.

10. A method according to one or more of the preceding claims, wherein the modification is specific for one type of amino acid, for instance an amine-residue and/or even is specific for only 1 amine-residue in the peptide or protein, said 1 amine for instance being the N- terminus.

11. A method according to any of the preceding claims wherein the substrate is human interleukin-3, said method preferably providing interleukin 3 modified only at one or more of the following residues: Ala¹, His²⁶, Lys²⁸, Lys⁶⁶, His⁹⁵, His⁹⁸, Lys¹⁰⁰, or Lys¹¹⁶.

12. A method according to any of the preceding claims for the introduction of an antagonistic and/or cell inhibitory activity said method comprising disruption of phosphate binding.

13. A modified signal substance with superagonistic or antagonistic activity selected from the group consisting of a growth factor, an interleukin, a colony stimulating factor a TNF and an interferon, wherein the modification is a chemical and/or molecular biological modification, within or in close proximity to a partial or complete catalytic center and said modification being within or in close proximity to a metal binding center and/or within a phosphate binding center.

14. A modified signal substance accoding to claim 13, wherein the metal binding center is a zinc binding center.

15. A modified signal substance accoding to claim 13 or 14, wherein the chemical modification is a gradual chemical modification.

16. A modified signal substance according to claims 13 to 15, wherein the modification is a deletion and/or substitution of an amino acid involved in the binding of a metal ion.

17. A modified signal substance according to claims 13 to 15, wherein the modification is an alkylation or an acylation.

18. A modified signal substance according to claims 13 to 17, wherein the modification is in a histidine in the metal binding center.

19. A modified signal substance according to claims 13 to 18, wherein the modified signal substance is an interleukin 1-8, GM-CSF, TNF or gamma-interferon.

20. Use of a modified signal substance according to claims 13 to 19 or a nucleic acid sequence encoding it for the preparation of a medicament for application in a treatment comprising stimulating stem cell replication or gene therapy.

## Patentansprüche

1. Verfahren zur quantitativen Strukturfunktionsanalyseforschung von biologisch aktiven Proteinen oder Peptiden, die aus humanen Rezeptoren wie z. B. Interleukinen, haemopoietischen Wachstumsfaktoren, Peptidhormonen oder Proteinhormonen, Signalpeptiden oder Signalproteinen zur Einführung von einer oder mehreren der folgenden Eigenschaften ausgewählt werden: Erhöhte biologische Aktivität, erhöhte Stabilität, unterdrückte Antigenität, erworbene antagonistische Aktivität oder zellinhibitorische Aktivität, genanntes Verfahren umfasst die Anwendung einer speziellen chemischen Abänderung von ausgewählten Aminosäuren unter Verwendung von
a) schrittweiser chemischer Abänderung des Proteins oder Peptids, gefolgt von
b) Kontrolle der Abänderungsreaktion mit einer milden und sensitiven Methode wie nicht-denaturierender Elektrophorese und/oder Elektrosprüh-Massenspektrometrie und genannte Kontrolle umfasst ferner wahlweise die Bestätigung der gesamten strukturellen Integrität, z.B. unter Verwendung der Zirkulardichroismus-Spektroskopie
c) Protease-Behandlung,
d) Massenspektrometrie und
e) Prüfung der biologischen Aktivität des abgeänderten Produkts und wahlweise einer Prüfung der Stabilität des abgeänderten Produkts, genannte Proteine oder Peptide werden wahlweise aus den Interleukinen 1 bis 8, dem Interleukin 10, GM-CSF, TNF, Insulin, Prolactin und gamma IFN, mehr bevorzugt GM-CSF, EPO oder einem Interleukin, das aus den Interleukinen 2 bis 7, ausgewählt aus der Zytokin-Superfamilie, ausgewählt wird, ausgewählt.

2. Verfahren gemäß Anspruch 1, worin spezifischer Verdau mit spezifischen Proteasen und Massenspektrometrie zur Charakterisierung und Lokalisation der abgeänderten Aminosäuren ausgeführt wird.

3. Verfahren gemäß Anspruch 1 oder 2, worin spezifischer Verdau mit spezifischen Endoproteasen und LDMS zur Charakterisierung und Lokalisation der abgeänderten Aminosäuren ausgeführt wird, genannte Endoprotease ist z. B. Endo Glu C oder Endo Lys C.

4. Verfahren gemäß irgendeinem der Ansprüche 1 bis 3, worin die Abänderung durch spezifischen Verdau mit spezifischen Exoproteasen ausgeführt wird und Elektorsprüh-Massenspektrometrie zur Charakterisierung und Lokalisation der abgeänderten Aminosäuren ausgeführt wird, geeigneter Weise ist die Exoprotease N-terminal, z. B. Cathepsin C, oder C-terminal, z. B. Carboxypeptidase Y.

5. Verfahren gemäß irgendeinem der Ansprüche 1 bis 3, worin die Abänderung eine chemische Abänderung ist, genannte Abänderung ist eine Alkylierung und/oder genannte Abänderung ist eine Acylierung, wie z. B. Acetylierung, z. B. durch Iodoacetat oder Succinylierung, z.B. durch Bernsteinsäureanhydrat, genannte Abänderung ist entsprechend eine Abänderung unter schrittweise variierenden Bedingungen, worin eine oder mehrere der folgenden Bedingungen wie folgt variiert werden: pH zwischen 5,0 und 7,0, vorzugsweise in Schritten von 0,5 pH-Einheiten, und/oder Zeit oder Reagenskonzentrationen werden variiert.

6. Verfahren gemäß Anspruch 5, worin die Abänderung in der Anwesenheit von Phosphatpuffer, vorzugsweise in Verbindung mit Acetanhydrid, ausgeführt wird.

7. Verfahren gemäß einem oder mehrerer der vorhergehenden Ansprüche zur Einführung einer antagonistischen und/oder zellinhibitorischen Aktivität, worin die Abänderung spezifische Wirksamkeit auf einen oder mehrere Reste, die an katalytischer Aktivität beteiligt sind, hat, z. B. worin die Abänderung innerhalb oder in enger Nachbarschaft zu einem teilweisen oder gesamten katalytischen Zentrum liegt, wobei genannte Abänderung vorzugsweise die katalytische Aktivität ändert, geeigneter Weise ist genannter Rest ein Histidinrest.

8. Verfahren gemäß irgendeinem der vorhergehenden Ansprüche, worin die Abänderung innerhalb oder in enger Nachbarschaft zu einem Metallbindungszentrum liegt, vorzugsweise ein Zinkbindungszentrum, geeigneter Weise ist genannter Rest ein Histidinrest.

9. Verfahren gemäß einem oder mehrerer der vorhergehenden Ansprüche, worin die Abänderung durch reversible Denaturierung des Substrats und durch Zugabe eines Chelatbildners zur Entfernung des Metallions, z. B. in der Anwesenheit von Harnstoff und EDTA durchgeführt wird, genannter Harnstoff hat vorzugsweise eine Konzentration, die höher ist als 5 M und genanntes EDTA hat vorzugsweise eine Konzentration von 50 mM.

10. Verfahren gemäß einem oder mehrerer der vorhergehenden Ansprüche, worin die Abänderung für eine Art von Aminosäure spezifisch ist, z. B. ein Aminrest, und/oder sogar für nur einen Aminrest in dem Peptid oder Protein spezifisch ist, genanntes Amin ist z. B. der N-Terminus.

11. Verfahren gemäß irgendeinem der vorhergehenden Ansprüche, worin das Substrat humanes Interleukin-3 ist, genanntes Verfahren stellt vorzugsweise Interleukin 3 bereit, das nur an einem oder mehreren der folgenden Reste abgeändert wurde: Ala¹, His²⁶, Lys²⁸, Lys⁶⁶, His⁹⁵, His⁹⁸, Lys¹⁰⁰, oder Lys¹¹⁶.

12. Verfahren gemäß irgendeinem der vorhergehenden Ansprüche zur Einführung einer antagonistischen und/oder zellinhibitorischen Aktivität, genanntes Verfahren umfasst die Zerrüttung des Bindens von Phosphat.

13. Abgeänderte Signalsubstanz mit superagonistischer oder antagonistischer Aktivität, ausgewählt aus der Gruppe, die aus einem Wachstumsfaktor, einem Interleukin, einem Kolonie-stimulierenden Faktor, einem TNF und einem Interferon besteht, worin die Abänderung eine chemische und/oder molekularbiologische Abänderung ist, innerhalb oder in enger Nachbarschaft zu einem teilweisen oder gesamten katalytischen Zentrum liegt, und genannte Abänderung liegt innerhalb oder in enger Nachbarschaft zu einem Metallbindungszentrum und/oder innerhalb eines Phosphatbindungszentrums.

14. Abgeänderte Signalsubstanz gemäß Anspruch 13, worin das Metallbindungszentrum ein Zinkbindungszentrum ist.

15. Abgeänderte Signalsubstanz gemäß Anspruch 13 oder 14, worin die chemische Abänderung eine schrittweise chemische Abänderung ist.

16. Abgeänderte Signalsubstanz gemäß den Ansprüchen 13 bis 15, worin die Abänderung eine Deletion und/oder Substitution einer Aminosäure ist, die an der Bindung eines Metallions beteiligt ist.

17. Abgeänderte Signalsubstanz gemäß den Ansprüchen 13 bis 15, worin die Abänderung eine Alkylierung oder eine Acylierung ist.

18. Abgeänderte Signalsubstanz gemäß den Ansprüchen 13 bis 17, worin sich die Abänderung in einem Histidin in dem Metallbindungszentrum befindet.

19. Abgeänderte Signalsubstanz gemäß den Ansprüchen 13 bis 18, worin die abgeänderte Signalsubstanz ein Interleukin 1 bis 8, GM-CSF, TNF oder gamma-Interferon ist.

20. Anwendung einer abgeänderten Signalsubstanz gemäß den Ansprüchen 13 bis 19 oder einer dafür kodierenden Nukleinsäuresequenz zur Zubereitung eines Arzneimittels für die Anwendung in einer Behandlung, die das Stimulieren der Stammzellreplikation oder Gentherapie umfasst.

## Revendications

1. Méthode de recherche d'analyse quantitative structure - fonction pour des protéines ou peptides biologiquement actifs choisis parmi des récepteurs humains tels que les interleukines, les facteurs de croissance hématopoïétiques, les hormones peptidiques ou les hormones protéiniques, les peptides signaux ou les protéines signaux, aux fins d'introduction d'une ou plusieurs des caractéristiques suivantes : activité biologique augmentée, stabilité augmentée, antigénicité supprimée, acquisition d'une activité antagoniste ou d'une activité inhibitrice cellulaire, ladite méthode comprenant l'application d'une modification chimique spécifique d'acides aminés sélectionnés avec utilisation
(a) d'une modification chimique graduelle de la protéine ou du peptide, suivie
(b) du suivi de la réaction modificatrice par une méthode douce et sensible telle qu'une électrophorèse non-dénaturante et/ou une spectrométrie de masse en mode électrospray et ledit suivi comprenant de façon optionnelle en outre la confirmation de l'intégrité de la structure globale, par exemple par utilisation de la spectroscopie de dichroïsme circulaire,
(c) d'un traitement par des protéases,
(d) de la spectrométrie de masse et
(e) de la détermination de l'activité biologique du produit modifié et, de façon optionnelle de la détermination de la stabilité du produit modifié, lesdits peptides ou protéines étant choisis, de préférence, parmi les interleukines 1 à 8, l'interleukine 10, GM-CSF, TNF, l'insuline, la prolactine et γIFN, plus préférablement GM-CSF, EPO ou une interleukine choisie parmi les interleukines 2 à 7 choisies dans la super famille des cytokines.

2. Méthode selon la revendication 1, dans laquelle une digestion spécifique avec des protéases spécifiques et une spectrométrie de masse sont effectuées pour caractériser et localiser les acides aminés modifiés.

3. Méthode selon la revendication 1 ou 2, dans laquelle une digestion spécifique avec des endoprotéases spécifiques et une spectrométrie de masse à désorption laser (LDMS) sont effectuées pour caractériser et localiser les acides aminés modifiés, ladite endoprotéase étant par exemple l'Endo Glu C ou l'Endo Lys C.

4. Méthode selon l'une quelconque des revendications 1 à 3, dans laquelle la modification est effectuée par digestion spécifique avec des exoprotéases spécifiques et la spectrométrie de masse en mode électrospray est effectuée pour caractériser et localiser les acides aminés modifiés, de façon appropriée l'exoprotéase est N-terminale, par exemple la cathepsine C, ou C-terminale, par exemple la carboxypeptidase Y.

5. Méthode selon l'une quelconque des revendications 1 à 3, dans laquelle la modification est une modification chimique, ladite modification étant une alkylation et/ou ladite modification étant une acylation, telle qu'une acétylation, par exemple par l'iodoacétate, ou une succinylation, par exemple par l'anhydride succinique, ladite modification étant, de façon appropriée, une modification dans des conditions variant graduellement, dans laquelle on fait varier une ou plusieurs des conditions suivantes comme suit : on fait varier le pH entre 5,0 et 7,0, de préférence par incréments de 0,5 unité de pH, et/ou la durée ou les concentrations des réactifs.

6. Méthode selon la revendication 5, dans laquelle la modification est effectuée en présence de tampon phosphate, de préférence en combinaison avec l'anhydride acétique.

7. Méthode selon l'une quelconque des revendications précédentes, pour l'introduction d'une activité antagoniste et/ou inhibitrice cellulaire, dans laquelle la modification est spécifique d'un ou plusieurs résidus impliqués dans l'activité catalytique, par exemple dans laquelle la modification est au niveau, ou très proche, d'un centre catalytique partiel ou complet, ladite modification modifiant de préférence l'activité catalytique, ledit résidu étant, de façon appropriée, un résidu histidine.

8. Méthode selon l'une quelconque des revendications précédentes, dans laquelle la modification est au niveau, ou très proche, d'un centre de liaison à un métal, de préférence un centre de liaison au zinc, ledit résidu étant, de façon appropriée, un résidu histidine.

9. Méthode selon l'une quelconque des revendications précédentes, dans laquelle la modification est réalisée par dénaturation réversible du substrat et par addition d'un agent chélatant pour éliminer l'ion métallique, par exemple en présence d'urée et d'EDTA, ladite urée étant de préférence à une concentration supérieure à 5 M, et ledit EDTA étant de préférence à une concentration de 50 mM.

10. Méthode selon l'une quelconque des revendications précédentes, dans laquelle la modification est spécifique d'un type d'acide aminé, par exemple un résidu amine et/ou est même spécifique d'uniquement un résidu amine dans le peptide ou la protéine, ladite amine étant par exemple la partie N-terminale.

11. Méthode selon l'une quelconque des revendications précédentes, dans laquelle le substrat est l'interleukine-3 humaine, ladite méthode donnant de préférence une interleukine-3 modifiée uniquement au niveau d'un ou plusieurs des résidus suivants : Ala¹, His²⁶, Lys²⁸, Lys⁶⁶, His⁹⁵, His⁹⁸, Lys¹⁰⁰ ou Lys¹¹⁶.

12. Méthode selon l'une quelconque des revendications précédentes, pour l'introduction d'une activité antagoniste et/ou inhibitrice cellulaire, ladite méthode comprenant la rupture d'une liaison phosphate.

13. Substance signal modifiée à activité superagoniste ou antagoniste choisie dans le groupe constitué par un facteur de croissance, une interleukine, un facteur stimulant les colonies, un TNF et un interféron, où la modification est une modification chimique et/ou de biologie moléculaire, au niveau ou très proche d'un centre catalytique partiel ou complet, ladite modification étant au niveau ou très proche d'un centre de liaison à un métal et/ou d'un centre de liaison à un phosphate.

14. Substance signal modifiée selon la revendication 13, dans laquelle le centre de liaison à un métal est un centre de liaison au zinc.

15. Substance signal modifiée selon la revendication 13 ou 14, dans laquelle la modification chimique est une modification chimique graduelle.

16. Substance signal modifiée selon les revendications 13 à 15, dans laquelle la modification est une délétion et/ou une substitution d'un acide aminé impliqué dans la liaison à un ion métallique.

17. Substance signal modifiée selon les revendications 13 à 15, dans laquelle la modification est une alkylation ou une acylation.

18. Substance signal modifiée selon les revendications 13 à 17, dans laquelle la modification sc situe dans une histidine dans le centre de liaison à un métal.

19. Substance signal modifiée selon les revendications 13 à 18, la substance signal modifiée étant une interleukine 1 à 8, GM-CSF, TNF ou le γ-interféron.

20. Utilisation d'une substance signal modifiée selon les revendications 13 à 19, ou d'une séquence d'acide nucléique codant pour celle-ci, pour la préparation d'un médicament destiné à être appliqué dans un traitement comprenant la stimulation de la réplication de cellules souches ou la thérapie génique.
